# EUROPEAN PATENT SPECIFICATION

(11) **EP 0 429 204 B1**
(45) Date of publication and mention of the grant of the patent: **10.08.1994**
(21) Application number: 90311989.9
(22) Date of filing: 01.11.1990
(51) Int. Cl.: A61B 17/39, A61B 1/04

(54) **Electrosurgery apparatus**
Elektrochirurgisches Gerät
Bistouri électrique

(30) Priority: 18.11.1989 GB 8926125
(43) Date of publication of application: 29.05.1991
(73) Proprietor: Smiths Industries Public Limited Company, London, NW11 8DS (GB)
(72) Inventor: TAYLOR, GEOFFREY PERCY, Findon Near Worthing, W Sussex, BN14 OSQ (GB)
(74) Representative: Flint, Jonathan McNeill

(56) References cited:
- EP-A- 0 299 240
- DE-A- 3 613 759
- US-A- 4 517 976

## Description

This invention relates to systems including electrosurgery apparatus and a video camera that provides an image of a surgical site on a display, the electrosurgery apparatus including a power supply unit that supplies electrosurgery pulses of radio frequency power to an active patient electrode, and a synchonisation unit having an input that receives a synchronisation pulse from the video camera.

Electrosurgery, such as diathermy, units are often used during surgery such as to cut tissue and coagulate blood vessels. Radio frequency current is supplied via a hand-held electrode that is applied to the surgical site and a return path is provided from the patient by means of a large area electrode fixed to some other part of the patient's body. Electrosurgery is particularly useful for operations on internal organs where access is restricted. Such operations are commonly carried out under observation by a television camera coupled to an endoscope, the camera's video output being supplied to a monitor by which progress of the operation can be viewed. One problem with this arrangement is that the electrosurgery output can interfere with the video output, especially in some modes, such as the spray coagulation mode. This can make observation very difficult, especially in view of the fact that the image on the monitor screen is generally confined to a circular region at the centre of the screen. Although it is possible to screen electrically the electrosurgery apparatus and the cables leading to the electrodes, it will be appreciated that it is inherent that the electrodes must be exposed so that they can be brought into contact with patient tissue, and also that there will be an unscreened path for electrical energy through the body. In US 4,517,976 there is described an arrangement in which electrosurgery pulses are primarily produced during the horizontal blanking period of the display. This avoids interference on the screen but can reduce the efficiency of the electrosurgery apparatus because of the restriction on the time during which pulses can be generated.

It is an object of the present invention to provide an improved system including electrosurgery apparatus by which the effects of interference on a video signal are reduced.

According to one aspect of the present invention there is provided a system of the above-specified kind, characterised in that the video camera produces the image in a central region of the display that has a width less than that of the display, and that the synchronisation unit synchronises the generation of the electrosurgery pulses to the camera output such that interference is produced in peripheral regions of the display representation provided by the camera but is absent from the central region of the display.

In this way, the effect of interference is minimized.

The image of the surgical site is preferably of a circular shape and the camera may be coupled with an endoscope. The system may include a visual display monitor, the video camera output being supplied to the monitor and the display representation being provided on a screen of the monitor. The system may include a video recorder, the output of the camera being supplied to the video recorder.

The synchronisation unit perferably synchronises the power supply unit to generate the pulses such that the longest interval between adjacent pulses occurs midway along a scan of the camera.

The apparatus may include a manual control, the manual control being adjustable to move the interference out of the field of interest of the display representation.

A system including electrosurgery apparatus and a video camera, in accordance with the present invention, will now be described, by way of example, with reference to the accompanying drawings, in which:
- Figure 1: shows the system schematically; and
- Figure 2: illustrates a display produced by an output of the camera.

The electrosurgery/diathermy apparatus is contained in a housing 1 and is connected to an active patient electrode 2 and a large area, return electrode 3. The housing 1 contains a power supply 10 which may be of conventional kind and includes the usual control and safety features to be found on such equipment. One example of a suitable power supply is described in GB 2225656. The housing 1 also includes a synchronisation unit 11 which controls the generation of pulses produced by the power supply. The synchronisation unit 11 is connected via a cable 12 to video monitoring apparatus 20 forming a part of the system.

The apparatus 20 comprises a conventional fibre-optic endoscope 21 having a television camera 22 coupled at its rear end which provides a video output on line 23 to a visual display monitor 24 having a screen 25. The video output can also be supplied to a recorder 26. The cable 12 is coupled to line 23 so that a sample of the video synchronisation pulses produced by the camera 22 are supplied to the sychronisation unit 11 in the electrosurgery housing 1. The periodicity of the video screen is 64 microseconds per line with approximately 52 microseconds appearing on the screen 25 of the monitor 24. The nature of the fibre-optic endoscope 21 is such that the image of the surgical site, as seen through the endoscope and as presented on the monitor 24, is a circular region in the centre of the monitor, the surrounding, peripheral region of the display representation being blank.

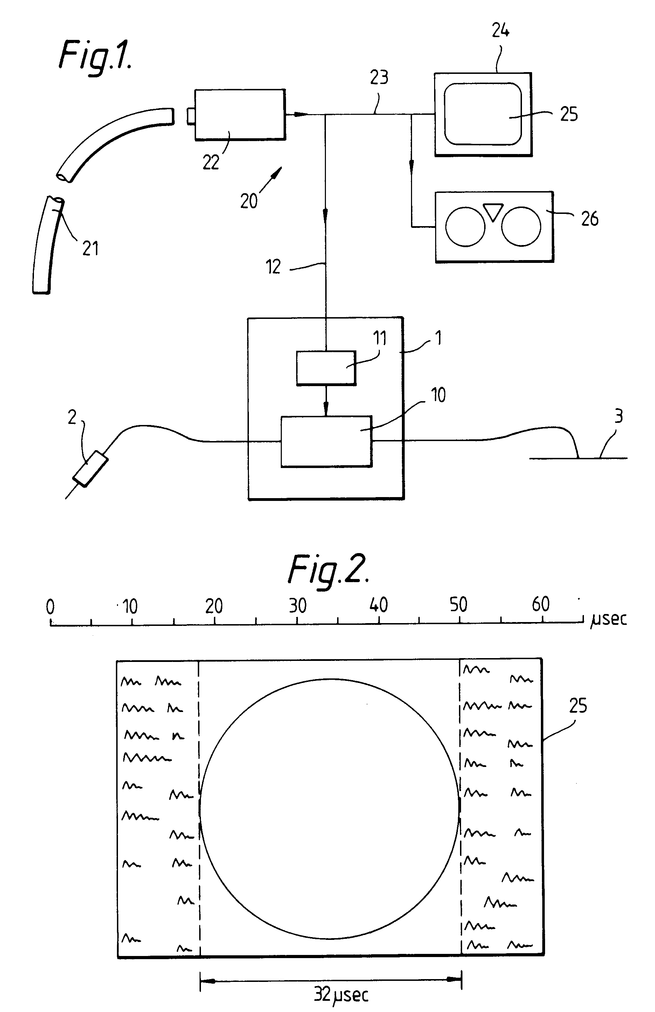

The synchronisation unit 11 is arranged to control the power supply 10 such that the electrosurgery pulses are only produced at times when the interference that they cause to the display is limited to the blank, peripheral regions and thereby avoids obscuring the central region in which the image of the surgical site is presented. More particularly, the synchronisation unit 11 synchronises the power supply unit 10 to generate the pulses when the longest interval between adjacent pulses, or groups of pulses occurs midway along a scan of the camera. For example, a normal spray coagulation output from electrosurgery apparatus would consist of sequence of single pulses with a fundamental frequency of 500kHz having a pulse separation of about 32 microseconds. The synchronisation unit 11 is arranged such that the pulses are produced at 18 microseconds and 50 microseconds along each line scan. The interference produced on the monitor will thereby be located at 12 microseconds along the scan line on the screen and at 44 microseconds along the scan line. In this way, the interference will be confined to two vertical bands of 12 microseconds width along opposite edges of the screen, leaving the central region, which is 32 microseconds wide, free of interference, as shown in Figure 2. Because the display representation is a circular image, occupying a central region of the screen, it can be seen that there will be little, if any interference in the region of interest.

It would also be possible for the synchronisation unit 11 to alter the spacing between pulses so that all the interference appears closer to the edge of the screen. For example, the electrosurgery pulses could be produced at locations of 10 microseconds and 54 microseconds along each scan line, so that they are unevenly spaced from one another but leave a central region, 44 microseconds wide, unoccupied by interference. The apparatus could be provided with a manual control which is adjusted by the user until the interference is moved out of the field of view of interest.

The arrangement of the present invention enables video equipment to be used with electrosurgery apparatus with a considerably improved visibility. The invention can also be used to improve the quality of video recordings made of electrosurgery procedures, such as for instructional purposes.

## Claims

1. A system including electrosurgery apparatus and a video camera (22) that provides an image of a surgical site on a display (25), the electrosurgery apparatus including a power supply unit (1) that supplies electrosurgery pulses of radio frequency power to an active patient electrode (2), and a synchronisation unit (11) having an input that receives a synchronisation pulse from the video camera (22), characterised in that the video camera (22) produces the image in a central region of the display (25) that has a width less than that of the display, and that the synchronisation unit (11) synchronises the generation of the electrosurgery pulses to the camera output such that interference is produced in peripheral regions of the display representation provided by the camera but is absent from the central region of the display.

2. A system according to Claim 1, characterised in that the image of the surgical site is of circular shape.

3. A system according to Claim 1 or 2, including an endoscope, characterised in that the camera (22) is coupled with an endoscope (21).

4. A system according to any one of the preceding claims, including a visual display monitor (24), that the video camera output is supplied to the monitor (24) and the display representation is provided on a screen (25) of the monitor (24).

5. A system according to any one of the preceding claims, including a video recorder, characterised in that the output of the video camera (22) is supplied to a video recorder (26).

6. A system according to any one of the preceding claims, characterised in that the sychronisation unit (11) synchronises the power supply unit (10) to generate the pulses such that the longest interval between adjacent pulses occurs midway along a scan of the camera (22).

7. A system according to any one of the preceding claims, characterised in that the apparatus includes a manual control, and that the manual control is adjustable to move the interference out of the field of interest of the display representation.

## Patentansprüche

1. System mit einem elektrochirurgischen Gerät und einer Videokamera (22), welche auf einer Anzeige (25) ein Bild der chirurgischen Eingriffsstelle erzeugt, wobei das elektrochirurgische Gerät eine Energieversorgungseinheit (1) umfaßt, die einer aktiven Patientenelektrode (2) Elektrochirurgiepulse im Radiofrequenzbereich zuführt, und mit einer Synchronisationseinheit (11) mit einem Eingang, welchem ein Synchronisationspuls von der Videokamera (22) zugeführt wird, **dadurch gekennzeichnet**, daß die Videokamera (22) das Bild in einem zentralen Bereich der Anzeige (25) erzeugt, dessen Breite geringer als die Breite der Anzeige ist und daß die Synchronisationseinheit (11) die Generierung von Elektrochirurgiepulsen auf eine Weise mit dem Kameraausgangssignal synchronisiert, auf welche nur in peripheren Bereichen der Anzeigedarstellung der Kamera Interferenzen erzeugt werden, nicht jedoch im mittleren Bereich der Anzeige.

2. System nach Anspruch 1, **dadurch gekennzeichnet**, daß das Bild der chirurgischen Eingriffsstelle kreisförmig ist.

3. System nach einem der Ansprüche 1 oder 2 mit einem Endoskop, **dadurch gekennzeichnet**, daß die Kamera (22) mit dem Endoskop (21) gekoppelt ist.

4. System nach einem der voranstehenden Ansprüche mit einem Anzeigemonitor (24), **dadurch gekennzeichnet**, daß das Ausgangssignal der Videokamera dem Monitor (24) zugeführt wird und die Anzeigedarstellung auf einem Bildschirm (25) des Monitors (24) erfolgt.

5. System nach einem der voranstehenden Ansprüche mit einem Videorecorder, **dadurch gekennzeichnet**, daß das Ausgangssignal der Videokamera (22) dem Videorecorder (26) zugeführt wird.

6. System nach einem der voranstehenden Ansprüche, **dadurch gekennzeichnet**, daß die Synchronisationseinheit (11) die Energieversorgungseinheit (10) zur Generierung von Pulsen synchronisiert, wobei der längste Abstand zwischen benachbarten Pulsen in der Mitte eines Zeilendurchgangs der Kamera (22) liegt.

7. System nach einem der voranstehenden Ansprüche, **dadurch gekennzeichnet**, daß die Vorrichtung eine manuelle Bedieneinheit umfaßt, mit welcher die Interferenzen aus dem interessierenden Bereich der Anzeigedarstellung hinausschiebbar sind.

## Revendications

1. Dispositif comportant un appareil électrochirurgical et une caméra vidéo (22) qui fournit une image d'un emplacement chirurgical sur un organe d'affichage (25), l'appareil électrochirurgical comportant une unité d'alimentation (1) qui délivre des impulsions d'électrochirurgie en radiofréquence à une électrode active (2) sur un patient, et une unité de synchronisation (11) ayant une entrée qui reçoit de la caméra vidéo (22) une impulsion de synchronisation, caractérisé en ce que la caméra vidéo (22) produit, dans une zone centrale de l'affichage (25), une image ayant une largeur moindre que celle de l'organe d'affichage et en ce que l'unité de synchronisation synchronise la production des impulsions d'électrochirurgie par rapport au signal de sortie de la caméra de telle sorte qu'une interférence est produite dans des zones périphériques de la représentation produite par la caméra sur l'organe d'affichage, mais absente de la zone centrale de l'affichage.

2. Dispositif selon la revendication 1, caractérisé en ce que l'image de l'emplacement chirurgical est de forme circulaire.

3. Dispositif selon la revendication 1 ou 2, comportant un endoscope, caractérisé en ce que la caméra (22) est couplée à un endoscope (21).

4. Dispositif selon l'une des revendications précédentes, comportant un moniteur d'affichage visuel (24), caractérisé en ce que le signal de sortie de la caméra vidéo est délivré au moniteur (24) et en ce que la représentation affichée est produite sur un écran (25) du moniteur (24).

5. Dispositif selon l'une des revendications précédentes, comportant un enregistreur vidéo, caractérisé en ce que le signal de sortie de la caméra vidéo (22) est délivré à un enregistreur vidéo (26).

6. Dispositif selon l'une des revendications précédentes, caractérisé en ce que l'unité de synchronisation (11) synchronise l'unité d'alimentation (10) pour que celle-ci délivre les impulsions de façon que le plus long intervalle entre des impulsions adjacentes se trouve au milieu d'une longueur de balayage de la caméra (22).

7. Dispositif selon l'une des revendications précédentes, caractérisé en ce que l'appareil comporte une commande manuelle et en ce que la commande manuelle est ajustable pour déplacer l'interférence hors du champ présentant un intérêt dans la représentation affichée.
